Europäisches Patentamt

European Patent Office

Office européen des brevets

(19)

(11) Publication number: **0 511 767 A1**

## EUROPEAN PATENT APPLICATION

(12)

(21) Application number: **92303526.5**

(22) Date of filing: **21.04.92**

(51) Int. Cl.⁵: **A61K 31/415**, A61K 9/20

(30) Priority: **29.04.91 US 692747**

(43) Date of publication of application:
**04.11.92 Bulletin 92/45**

(84) Designated Contracting States:
**AT BE CH DE DK ES FR GB GR IT LI LU NL PT SE**

(71) Applicant: **MERCK & CO. INC.**
**126, East Lincoln Avenue P.O. Box 2000**
**Rahway New Jersey 07065-0900(US)**

(72) Inventor: **Katdare, Ashok V.**
**215 W. Freedley Street**
**Norristown, PA 19401(US)**
Inventor: **Cunningham, John C.**
**1158 W. Main Street, Apt.D1-23**
**Lansdale, PA 19446(US)**

(74) Representative: **Thompson, John Dr. et al**
**Merck & Co., Inc. European Patent**
**Department Terlings Park Eastwick Road**
**Harlow, Essex CM20 2OR(GB)**

(54) **Tablets containing compound DUP753.**

(57) An optimized direct compression tablet formulation comprises in parts by weight from about 10% to about 45% of 2-butyl-4-chloro-1[(2'(1H-tetrazol-5-yl)biphenyl-4-yl)methyl]-5-(hydroxymethyl)-imidazole, from about 20% to about 40% microcrystalline cellulose, from about 10% to about 30% lactose, from about 0.5% to about 0.9% magnesium stearate, and from about 5% to about 35% pregel starch.

EP 0 511 767 A1

BACKGROUND OF THE INVENTION

As is known from the prior art, e.g., published European Patent Application 0 253 310, 2-butyl-4-chloro-1[(2'(1H-tetrazol-5-yl)biphenyl-4-yl)methyl]-5-(hydroxymethyl)imidazole, hereafter called DUP 753, inhibits the action of the hormone angiotensin II (AII) and is useful therefore in alleviating angiotensin induced hypertension. The enzyme renin acts on a blood plasma $\alpha_2$-globulin, angiotensinogen, to produce angiotensin I, which is then converted by angiotensin converting-enzyme to AII. The latter substance is a powerful vasopressor agent which has been implicated as a causative agent for producing high blood pressure in various mammalian species, such as the rat, dog, and man. DUP 753 inhibits the action of AII at its receptors on target cells and thus prevents the increase in blood pressure produced by this hormone-receptor interaction. By administering DUP 753 to a species of mammal with hypertension due to AII, blood pressure is reduced. DUP 753 also is useful for the treatment of congestive heart failure.

European Patent Application 0 253 310 discloses the following tablet formulation for DUP 753:

| Ingredient | Quantity (mg) | % (wt) |
|---|---|---|
| DUP 753 | 100 | 20.41 |
| Colloidal SiO$_2$ | 0.2 | 0.04 |
| Magnesium stearate | 5 | 1.02 |
| Microcrystalline cellulose | 275 | 56.12 |
| Starch | 11 | 2.25 |
| Lactose | 98.8 | 20.16 |

OBJECTS OF THE INVENTION

It is an object of the present invention to provide an optimized direct compression tablet formulation containing DUP 753 as active ingredient. Another object is to provide an optimized submultiple formulation containing the active ingredient of the present invention. A further object is to provide a DUP 753 tablet formulation having sufficient mechanical strength to permit film coating. These and other objects of the present invention will be apparent from the following description.

SUMMARY OF THE INVENTION

An optimized direct compression tablet formulation containing DUP 753 as active ingredient comprises in parts by weight from about 20% to about 40% microcrystalline cellulose, from about 10% to about 30% lactose, from about 0.5% to about 0.9% magnesium stearate, from about 5% to about 35% pregel starch, and from about 10% to about 45% of DUP 753.

DETAILED DESCRIPTION

In developing a new tablet dosage formulation, it is necessary to balance the often-competing needs of the clinical, marketing and production areas. In order to properly do so:

1. Each tablet much be uniform in weight and contain the appropriate amount of active ingredient. The flowability of the formulation is critical.

2. The active component of each tablet must be readily available as needed; hence the tablets must have the proper type of drug release and the appropriate dissolution characteristics. The solubility of the drug is critical.

3. The tablet formulation must be physically and chemically stable. Proper choice of method of manufacture and selection of excipients is critical.

4. The tablets must have the mechanical integrity to withstand damage during manufacturing, packaging, shipping and use.

5. The chosen method of manufacture must be efficient, reproducible, and amenable to automation.

6. The tablets must be elegant in appearance and aesthetically pleasing.

The present invention relates to an optimized direct compression tablet formulation containing DUP 753 as active ingredient.

Depending upon the properties of the active component, some of the objectives may assume more importance over others. For example, in the case of DUP 753, where an immediate release dosage form is required, the proper dissolution specification is not difficult to achieve due to the inherently high water solubility of this drug. However, since the tablet will need to be film coated to mask its bitter taste, the tablets must have sufficient mechanical strength to withstand this additional, often-abrasive processing step.

Often when a new drug entity is in early development, the clinical research group will request a dosage form with a certain type of drug release, e.g., immediate, sustained, or controlled release, along with a particular dosage range, although doses become well defined only approximately 6-9 months into a program. The rising dose study gives some early indication of what doses may be pharmacologically active and physiologically viable. As the clinical research group conducts Phase I studies, the proposed dosage range may be revised. Concurrently with these Phase I studies, the development of a formula is underway. Since the dosage level can dramatically affect the tablet composition, the pharmacist must develop a formula that is flexible and amenable to any revision in the dosage level. An excellent approach is to use submultiples which is described below.

Submultiples means keeping the relative composition of the tablet unchanged and only changing the weight of the tablet to contain a desired dose. For example, for a 20% drug composition:

| dose (mg) | Tablet Wt. (mg) |
|-----------|-----------------|
| 10 | 50 |
| 20 | 100 |
| 40 | 200 |
| 80 | 400 |

This approach will:

1. keep the development work to a minimum;

2. provide flexibility in changing doses without changing the compositions;

3. reduce stability, analytical, bioequivalence, bioavailability, formula characterization and administrative work significantly; and

4. require much smaller bulk supplies during formulation development compared to the situation when different doses have different compositions.

The submultiple approach is useful except when the dose ranges are uncomfortably wide such as, for example, 1 mg to 400 mg. However, if they are manageably wide such as 2.5 - 80 mg, it is still possible to use this approach by using two submultiples: one for 2.5 (50 mg tablet), 5 (100), and 10 (200 mg) and one for 20 mg (100 mg tablet), 40 (200 mg), and 80 (400 mg). This approach still reduces the workload significantly.

Direct compression was selected as the method of manufacture bacause it is much more economical, much less labor-intensive and easier to characterize than other tableting methods, and the specific properties of DUP 753 show that it is suitable for direct compression:

1. DUP 753 exhibits acceptable flow properties and does not have a tendency to agglomerate or segregate due to static charge.

2. While the compactibility of DUP 753 decreases as its % in the tablet increases, sufficient compactibility is obtainable at acceptable drug loading levels.

3. The percentage of drug in the tablet is sufficiently high that content uniformity in the direct compression mixtures is not a problem.

4. Uniformity of bulk drug and excipient properties can be controlled to produce direct compression tablets reproducibly and successfully.

The percent of DUP 753 will depend in part on the drug loading profile and the dosing requirements. As a part of additional preformulation work relevant to tablet development, we evaluated the effect of % drug loading on compaction properties and in turn tablet properties such as breaking strength and friability. We evaluated drug loads of 0%, 10%, and 50%. The results showed that as the % drug load increased, the breaking strength decreased. The breaking strength values at 50% drug load were unacceptable. Since DUP 753 is very bitter it requires film coating. Development of a strong tablet was set as a major objective to obtain a sufficiently hard tablet that can withstand film coating.

As development work progressed, it became evident that the clinically effective doses would be in the range of from about 50 mg to about 200 mg.

Since it was previously determined that 50% drug loading results in unacceptable compactibility, 33% drug loading was chosen. The 33% drug loading gives an elegantly acceptable size tablet over the 50 - 200 mg dose range.

50 mg = 150 mg tablet

100 mg = 300 mg tablet

200 mg = 600 mg tablet

Excipient selection depends on various factors, such as, the choice of DUP 753 percent, the objectives of the tablet formulation development, and the method of manufacture. The foremost property an excipient must possess is compatibility with DUP 753. The requirement for a hard, nonfriable tablet that could withstand film coating, necessitated choice of other excipients with excellent compactibility. Microcrystalline cellulose was found to have excellent compactibility with DUP 753. In addition, microcrystalline cellulose has a bulk density close to that of DUP 753 (0.3 - 0.4 g/ml) which may prevent segregation. Microcrystalline cellulose also acts as a wicking agent by virtue of its structure and promotes disintegration. Although in this particular case disintegration/dissolution is not a main concern since the drug is highly soluble, microcrystalline cellulose has been found to promote good adherence of film coat and formation of a sufficiently hard, nonfriable DUP 753 tablet. Various grades such as, for example, Avicel PH 101, Avicel PH 102 and Emcocell are suitable

Magnesium stearate was found to be compatible with DUP 753 and it was also selected as an excipient as it is an excellent lubricant. Its surface area and particle shape are of utmost importance in its function as a lubricant. Although magnesium stearate is generally a very small proportion of the total tablet, it is believed to be an extremely important and essential variable. Other materials that can be substituted in whole or in part for magnesium stearate are, for example, stearic acid, calcium stearate, sodium stearyl fumarate and talc.

A disadvantage of microcrystalline cellulose is believed to be its susceptibility to overlubrication by magnesium stearate. Overlubrication can occur due to too much magnesium stearate or too long a lubrication time. Magnesium stearate covers the surface of particles or granules during the lubrication step. This surface magnesium stearate can dramatically reduce the strength of tablets by interfering with bonding between particles or granules during compaction. To prevent this overlubrication, lactose was added. Besides being compatible with DUP 753, lactose compacts by brittle fracture. Lactose particles fracture to generate new unadulterated surfaces that may not only promote bonding but also reduce the risk of overlubrication since magnesium stearate does not cover the new fractured surfaces. Lactose is soluble, moisture insensitive, erodes easily in aqueous media, and available worldwide. It was believed that the inclusion of lactose also will improve the bulk density of the powder mixture which in turn should improve die filling at higher speeds. By way of illustration, examples of suitable lactoses are anhydrous lactose, fast flow lactose and spray dried lactose.

To further improve the bulk density requirement, pregel starch was included. Besides having good flow properties, it was shown to be compatible with DUP 753, possesses higher bulk density, is easily available worldwide and is known to have a good quality control. Pregelatinized (pregel) starches are prepared by cooking and drying starch slurries. The precooking process swells the starch granules and preconditions them so that they will thicken and gelatinize in cold liquids without the need for subsequent heating. By way of illustration, a specific example of pregel starch is NF 1500 (StaRx).

Since the relative proportions of the active and the excipients were believed to affect the final tablet properties, a mixture type of design naturally evolved. One type of mixture design is referred to as a slack variable design. In a slack variable design, one of the factors, called the slack or filler variable, is allowed to vary such that the % of the remaining factors + the % of the filler variable equals 100% of a fixed total. A slack variable design was used to optimize the DUP 753 formulation as the upper and lower bounds on the factor ranges satisfied the requirements for a slack variable design.

Pregel starch, which serves as a disintegrant, was believed to be the least important contributor to a formulation containing such a highly soluble active as DUP 753 and, therefore, it was selected as the filler.

In addition to selecting pregel starch as the filler, it was decided to fix the relative amount of DUP 753 at 33.33%. As earlier stated, this has a few important advantages: (1) compactibility problems due to high drug loading (>33.33%) are avoided; (2) the submultiple approach will work easily in the proposed dose range of 50-200 mg; and (3) interpretation of the results should be less difficult since one very important factor, % active, is kept constant for all dosage formulations.

After selecting pregel starch as the filler and deciding to keep the relative amount of active to 33.33%, the following ranges were selected for initial evaluation:

| | Microcrystalline Cellulose | Lactose | Magnesium Stearate | Starch | DUP 753 |
|---|---|---|---|---|---|
| Low | 20% | 10% | 0.3% | Filler to | 33.33% |
| High | 35% | 30% | 1.2% | 100% | 33.33% |

When compositions within the foregoing ranges were tested for feasibility, the two worst formulations were identified:

1. 20% microcrystalline cellulose, 30% lactose, 0.3% magnesium stearate, 16.37% starch, and 33.33% DUP 753; due to binding caused by underlubrication.

20% microcrystalline cellulose, 10% lactose, 1.2% magnesium stearate, 35.47% starch, 33.33% DUP 753; due to poor compactibility caused by overlubrication.

Both formulations proved to be nonfeasible. This finding necessitated constricting the range of magnesium stearate. Therefore, studies were conducted wherein the range of this ingredient ranged from a low of about 0.5% to a high of about 0.9%.

The formulation found to have the highest tensile strength/applied force (TS/AF) ratio was composed of 35% microcrystalline cellulose, 17.5% lactose, 0.8% magnesium stearate, 13.37% starch and 33.33% DUP 753. To minimize the potential for over-lubrication, further experiments were conducted having a narrower range of magnesium stearate. These studies resulted in a formulation composed of 35% microcrystalline cellulose, 17.0% lactose, 0.7% magnesium stearate, 13.97% starch and 33.33% DUP 753.

The following examples illustrate the present invention without, however, limiting the same thereto. While specific techniques are described therein for preparing direct compression tablets it is to be understood that other techniques may be employed as well.

EXAMPLE 1

A direct compression composition according to the present invention is prepared in a 250 g batch from the following ingredients.

|  | % | Grams |
|---|---|---|
| Microcrystalline cellulose (Avicel PH 102) | 35.0 | 87.5 |
| Lactose | 17.5 | 43.75 |
| Magnesium stearate | 0.8 | 2.0 |
| Pregel starch NF 1500 (StaRx) | 13.37 | 33.42 |
| DUP 753 | 33.33 | 83.33 |

The foregoing formulation was prepared for tablet manufacture by the following process. The microcrystalline cellulose, DUP 753, lactose and pregel starch were added to a V-blender having a size of approximately 1 liter, mixed for 5 minutes at a rotational speed of 24 rpm, and screened through a 30-mesh (US) screen. The mixed powder was re-mixed for an additional 5 minutes at the same rotational speed of 24 rpm and then spread onto tray paper. The magnesium stearate was manually forced through a 60-mesh (US) screen on top of the powder on the paper. The magnesium stearate then was worked gently into the mixed powder, and the resulting mixture blended in the V-blender for 4 minutes at 24 rpm. The blended powder was stored in a polyethylene bag until compaction.

Tablets with a final weight of 100 mg were compacted on the instrumented Stokes F press using flat, round, 0.25 inch (0.64 cm) tooling. The tablets had a TS/AF ratio of 257.1 kPa/kN, and were eminently suited for film coating.

EXAMPLE 2

A direct compression composition according to the present invention is prepared in a 250 g batch from the following ingredients.

|  | % | Grams |
|---|---|---|
| Microcrystalline cellulose (Avicel PH 102) | 35.0 | 87.5 |
| Lactose | 17.0 | 42.5 |
| Magnesium stearate | 0.7 | 1.75 |
| Pregel starch NF 1500 (StaRx) | 13.97 | 34.92 |
| DUP 753 | 33.33 | 83.33 |

The foregoing formulation was prepared following the procedure of example 1.

## EXAMPLES 3-12

The following Table shows various additional acceptable formulations of the present invention wherein all ingredients are varied in quantity except DUP 753 which is maintained at 33.3%.

| | Avicel (%) | Lactose (%) | Mag. St. (%) | StaRx (%) | Disltn.[1] (% diss/min) | TS/AF (kPa/kN) | Wt. (%) | Friability (%) | ESS[2] (N/cm2) | Disltn. (seconds) |
|---|---|---|---|---|---|---|---|---|---|---|
| 3 | 20.00 | 10.00 | 0.50 | 36.17 | 6.45 | 144 | 1.16 | 0.91 | 641 | 420 |
| 4 | 20.00 | 10.00 | 0.80 | 35.87 | 6.23 | 95 | 1.09 | 0.77 | 605 | 406 |
| 5 | 20.00 | 30.00 | 0.50 | 16.17 | 6.27 | 103 | 0.95 | 0.87 | 1301 | 382 |
| 6 | 20.00 | 30.00 | 0.80 | 15.87 | 6.16 | 165 | 1.22 | 0.64 | 1026 | 447 |
| 7 | 35.00 | 10.00 | 0.50 | 21.17 | 5.68 | 229 | 0.81 | 0.80 | 862 | 432 |
| 8 | 35.00 | 10.00 | 0.80 | 20.87 | 5.92 | 225 | 0.89 | 0.85 | 833 | 478 |
| 9 | 35.00 | 30.00 | 0.50 | 1.17 | 3.22 | 248 | 1.20 | 0.59 | 1204 | 501 |
| 10 | 35.00 | 30.00 | 0.80 | 0.87 | 3.31 | 299 | 0.82 | 0.59 | 1115 | 500 |
| 11 | 27.50 | 20.00 | 0.65 | 18.52 | 5.92 | 212 | 0.90 | 0.56 | 752 | 446 |
| 12 | 27.50 | 20.00 | 0.65 | 18.52 | 5.66 | 214 | 0.65[a] | 0.55 | 764 | 436 |

Fixed Factors: DRUG = 33.33

[a] Due to technical difficulties, weight variation for this run was excluded from the analysis

[1] Disltn. (% diss./min): A dissolution rate at least of 5% dissolved/min. is desirable.

[2] ESS = $\dfrac{\text{Ejection Force}}{\text{Contact Area}} = EF \int dt$

## Claims

1. A formulation suitable for forming a direct compression tablet comprising in parts by weight from about 20% to about 40% microcrystalline cellulose, from about 10% to about 30% lactose, from about 0.5% to about 0.9% stearic acid, magnesium or calcium stearate, sodium stearyl fumarate or talc from about 5% to about 35% pregel starch, and from about 10% to about 45% 2-butyl-4-chloro-1-[(2'(1H-tetrazol-5-yl)biphenyl-4-yl)methyl]-5-hydroxymethyl)-imidazole.

2. A tablet formed from a formulation according to claim 1.

3. A formulation according to claim 1 wherein the amount of 2-butyl-4-chloro-1-[(2'(1H-tetrazol-5-yl)-biphenyl-4-yl)methyl]-5-hydroxymethyl)-imidazole is about 33.33%.

4. A tablet formed from a formulation according to claim 3.

5. A formulation according to claim 3 containing about 35% microcrystalline cellulose, from about 17% to about 17.5% lactose, from about 0.7% to about 0.8% magnesium stearate from about 13.37 to about 13.97 pregel starch, and about 33.33% 2-butyl-4-chloro-1-[(2'(1H- tetrazol- 5-yl)biphenyl-4-yl)methyl]-5-hydroxymethyl)imidazole.

6. A tablet formed from a formulation according to claim 5.

7. A composition according to claim 5 containing about 35% microcrystalline cellulose, about 17.5% lactose, about 0.8% magnesium stearate, about 13.37% pregel starch and about 33.33% 2-butyl-4-chloro-1-[(2'(1H- tetrazol- 5-yl)biphenyl-4-yl)-methyl]-5-hydroxymethyl)imidazole.

8. A tablet formed from a formulation according to claim 7.

9. A composition according to claim 5 containing about 35% microcrystaline cellulose, about 17% lactose, about 0.7% magnesium stearate, about 13.97% pregel starch and about 33.33% 2-butyl-4-chloro-1-[(2'-(1H- tetrazol- 5-yl)biphenyl- 4-yl)-methyl]-5-hydroxymethyl)imidazole.

10. A tablet formed from a formulation according to claim 9.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| D,X | EP-A-0 253 310 (DU PONT)<br>* Claims 1,5; page 288: "Tablets" *<br>----- | 1-2 | A 61 K 31/415<br>A 61 K 9/20 |

TECHNICAL FIELDS
SEARCHED (Int. Cl.5)

A 61 K

The present search report has been drawn up for all claims

| Place of search | Date of completion of the search | Examiner |
|---|---|---|
| THE HAGUE | 22-06-1992 | SCARPONI U. |